Europäisches Patentamt

European Patent Office

Office européen des brevets

(11) Veröffentlichungsnummer: **0 155 505**
**B1**

(12) # EUROPÄISCHE PATENTSCHRIFT

(45) Veröffentlichungstag der Patentschrift:
28.06.89

(21) Anmeldenummer: 85101617.0

(22) Anmeldetag: 14.02.85

(51) Int. Cl.⁴: **A 61 L   2/18**, A 01 N 59/00,
G 02 C 13/00

(54) Kontaktlinsenpflegesatz und Verfahren zur Pflege von Kontaktlinsen sowie ein Reinigungsgerät hierfür.

(30) Priorität: 21.03.84 DE 3410400

(43) Veröffentlichungstag der Anmeldung:
25.09.85 Patentblatt 85/39

(45) Bekanntmachung des Hinweises auf die Patenterteilung: 28.06.89 Patentblatt 89/26

(84) Benannte Vertragsstaaten:
AT BE CH DE FR GB IT LI LU NL SE

(56) Entgegenhaltungen:
EP--A-- 0 082 798
EP--A-- 0 083 820
EP--A-- 0 110 609
EP--A-- 0 142 623
FR--A-- 2 134 666
GB--A-- 1 340 516
GB--A-- 1 500 707
US--A-- 3 623 492
US--A-- 4 414 127

(73) Patentinhaber: CIBA-GEIGY AG
Klybeckstrasse 141
CH-4002 Basel (CH)

(72) Erfinder: Ludwig, Gerhard, Dr. Dipl.-Chem.
Stromberger Strasse 24
D-6531 Weiler (DE)
Erfinder: Sunderdiek, Rainer, Dr.
Unterhainstrasse 19
D-8750 Aschaffenburg-Schweinheim (DE)
Erfinder: Hombach, Hans-Georg
Am Tiefen Weg 16
D-8786 Karlstadt (DE)
Erfinder: Steinburg, Heinz
Ostend 16
D-5870 Hemer (DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft einen Kontaktlinsenpflegesatz nach dem Oberbegriff des Anspruchs 1, ein Verfahren zur Pflege von Kontaktlinsen nach dem Oberbegriff des Anspruchs 9 sowie ein Reinigungsgerät nach dem Oberbegriff des Anspruchs 11.

Ein derartiger Kontaktlinsenpflegesatz und ein entsprechendes Pflegeverfahren sind aus der EP-A-0 082 798 bekannt.

Pflegesätze für weiche und harte Kontaktlinsen dienen zur Reinigung, Desinfizierung, Stabilisierung der optischen Eigenschaften und Verbesserung des Tragekomforts der Kontaktlinsen. Für die Desinfektion und Reinigung von Kontaktlinsen hat das Wasserstoffperoxid als antimikrobielle Substanz in neuerer Zeit Bedeutung gewonnen. Wasserstoffperoxid besitzt gegenüber Chlorhexidinsalzen oder quecksilberorganischen Verbindungen, z. B. Thiomersal, welche ebenfalls zur Desinfektion von Kontaktlinsen verwendet werden, eine bessere antimikrobiologische Wirkung, insbesondere bezüglich der relativ resistenten Aspergillosarten. Die bekannten, bei der Reinigung von Kontaktlinsen verwendeten Wasserstoffperoxidlösungen sind dreiprozentige Peroxidlösungen. Hieraus resultiert, daß nach der Behandlung der Kontaktlinsen mit der Wasserstoffperoxidlösung relativ große Restmengen an der Linse haften, die nach dem Einsetzen zu mehr oder weniger starken Schleimhautreizungen führen. Es ist daher erforderlich, nach der Behandlung mit einer Wasserstoffperoxidlösung die Linse mit einer Neutralisationslösung zu behandeln, durch die das Wasserstoffperoxid in Wasser und Sauerstoff aufgespalten wird. Hierzu ist es bekannt, Metallkatalysatoren wie Platin, Palladium oder organische Katalysatoren wie Katalasen bzw. Peroxidasen (Enzyme), organische Schwefelverbindungen oder Sulfide zu verwenden. Von diesen empfiehlt sich die oxydative enzymatische Peroxidzerstörung. Zugesetzte Reduktionsmittel verbleiben nämlich in der Lösung und beeinflussen deren Tonizität, so daß unter Umständen zusätzliche Spülschritte erforderlich sind.

Aufgabe der Erfindung ist es daher, einen Kontaktlinsenpflegesatz sowie ein Verfahren zur Kontaktlinsenpflege. und ein hierfür geeignetes Reinigungsgerät zu schaffen, durch welche eine Verringerung der Konzentration an Peroxid bei der Desinfektion und Reinigung der Kontaktlinsen und die sichere Beseitigung von nach der Desinfektions- und Reinigungsbehandlung an der Kontaktlinse haftenden Restmengen an Wasserstoffperoxid zumindest auf eine tolerierbare Menge erzielt wird.

Zur Lösung dieser Aufgabe wird durch die Erfindung ein Kontaktlinsenpflegesatz geschaffen, der einen Lösungsmittelsatz, bestehend aus zwei Lösungen, aufweist, von denen die eine Lösung eine Wasserstoffperoxidlösung und die andere eine enzymhaltige Neutralisationslösung ist. Die Wasserstoffperoxidlösung besitzt im Vergleich zu bekannten zur Pflege von Kontaktlinsen verwendeten Wasserstoffperoxidlösungen eine niedrige Konzentration (0,5-3,0 %), die vorzugsweise 0,5 bis 2,0 % oder bis 1,5 % beträgt. Es hat sich herausgestellt, daß die Abtötung von Mikroorganismen durch die gering konzentrierte Wasserstoffperoxidlösung (bis herunter zu 0,5 %) keineswegs langsamer und unsicherer verläuft. Bei den Keimen Pseudomonas aeruginosa und Escherichia coli erfolgt die Abtötung praktisch in der gleichen Zeit wie bei einer höherprozentigen Lösung. Bei den Keimen Candida albicans und Aspergillus niger erweist sich für die Abtötung eine Zeit von zwei Stunden als ausreichend.

Die Wasserstoffperoxidlösung enthält außerdem Zusätze, die die Ablösung von auf der Linse gebundenem Protein während der Einlagerungszeit verstärken. Dies ist insbesondere für die. Reinigung von weichen Kontaktlinsen von Bedeutung. Eine ähnliche Wirkung kann auch bei der Lipoidentfernung von harten Kontaktlinsen erzielt werden.

Da die Einlagerungszeit der Linse in der Wasserstoffperoxidlösung wesentlich das Ausmaß der Proteinreinigung beeinflußt, empfiehlt es sich, die Linse, insbesondere Weichlinse, während der ganzen Nacht in der Wasserstoffperoxidlösung zu belassen.

Durch die verringerte Konzentration (Verringerung bis auf etwa ein Sechstel gegenüber bekannten Wasserstoffperoxidlösungen für die Kontaktlinsenreinigung) ergeben sich geringere Restmengen von Wasserstoffperoxid an der behandelten Linse. Durch die geringere Restmenge ergibt sich bei der sich anschließenden Neutralisationsbehandlung eine sichere Entfernung des an der Kontaktlinse haftenden Wasserstoffperoxids. Es genügt eine Neutralisationsbehandlung von etwa 3 bis 5 Minuten, um die noch anhaftende Wasserstoffperoxidmenge auf eine zulässige Restmenge abzubauen. Ein absolut sicherer Abbau des Restwasserstoffperoxids wird bei einer Neutralisationsbehandlung von 10 Minuten erreicht.

Durch die verringerte Konzentration der Wasserstoffperoxidlösung wird auch das Kontaktlinsenmaterial weniger angegriffen als bei den herkömmlichen dreiprozentigen Reinigungslösungen.

Die Neutralisationslösung enthält oberflächenaktive benetzungsfördernde Substanzen, die im Gegensatz zu bislang gebräuchlichen Substanzen für Enzyme, insbesondere Katalasen, kompatibel sind. Es wurde überraschenderweise sogar gefunden, daß die Stabilität des in der Neutralisationslösung verwendeten Enzyms (Katalasen) erhöht wird.

Die Neutralisationslösung kann frei von Konservierungsstoffen gehalten werden, für die neuerdings Thiomersal, Sorbinsäure und ähnliche vorgeschlagen werden und welche in Lösung sehr labil sind und bei denen die benötigten Konzentrationen mit mindestens 0,1 % relativ hoch sind.

Die bei der Erfindung zur Anwendung kommende Neutralisationslösung zeichnet sich dadurch aus, daß sie eine physiologisch neutrale Salzlösung ist, Konservierungsstoffe nicht unbedingt aufweisen muß, eine Benetzungskomponente aufweist, die insbesondere bei der Behandlung von harten Kontaktlinsen von Bedeutung ist, durch den niedrigen Enzymanteil zu einem schnellen Wasserstoffperoxidabbau, d. h. Aufspaltung des Wasserstoffperoxids in Wasser und Sauerstoff, führt und eine enzymstabilisierende Wirkung besitzt.

Vorzugsweise kommt als weiterer Bestandteil neben dem Pflegemittelsatz beim Kontaktlinsenpflegesatz ein Reinigungsgerät zur Anwendung, durch das eine einfache und sichere Handhabung sowie reproduzierbare und Linsenmaterial schonende Wirkung bei der Durchführung des Reinigungs- und Neutralisationsschrittes gewährleistet wird.

Moderne Kontaktlinsen sind in den vergangenen Jahren aus Verträglichkeitsgründen zunehmend mit immer geringeren Mittendicken hergestellt worden. Die seit einiger Zeit stärker im Gespräch befindlichen Weichlinsen-Materialien mit höherem Wassergehalt weisen eine deutlich geringere mechanische Festigkeit gegenüber herkömmlichen Weichlinsenmaterialien auf.

Zudem kamen neue Hartlinsen-Materialien auf den Markt, die eine mechanisch relativ empfindliche Oberfläche aufweisen.

Die vorzugsweise zusammen mit dem Kontaktlinsenpflegesatz zur Reinigung der Kontaktlinsen vorgeschlagene Anwendung eines speziellen Reinigungsgeräts trägt diesen Schwachstellen konventioneller Linsenpflege durch Standardisierung des mechanischen Reinigungsprozesses Rechnung.

Bei diesem im Anspruch 11 angegebenen Reinigungsgerät ist gegenüber dem aus der US-A-36 23 492 bekannten Reinigungsgerät die Verwendung zweier Pflegemittellösungen, die nacheinander auf die Kontaktlinse zur Einwirkung gebracht werden, erleichtert. Dies wird dadurch erreicht, daß auf dem Deckel, der aus zwei gegeneinander verdrehbaren Deckelteilen besteht, Markierungen vorgesehen sind. Die beiden Deckelteile können nach dem ersten Behandlungsschritt, während welchem die Wasserstoffperoxidlösung auf die Kontaktlinse zur Einwirkung gebracht wird, je eine Markierung auf den beiden Deckelteilen einander gegenüber liegen, wodurch die Handhabung durch den Benutzer vereinfacht wird, weil durch diese Stellung angegeben wird, daß der erste Behandlungsschritt durchgeführt ist. Auf diese Weise wird eine Verwechslung der beiden Behandlungsschritte vermieden.

Außerdem wird die Handhabung beim Einlegen der Kontaktlinsen in das Reinigungsgerät erleichtert durch die gelenkige Anordnung des Kontaktlinsenträgers, weil hierdurch der Teil des Kontaktlinsenträgers, z. B. der mittlere Trägerteil, jeweils horizontal angeordnet bzw. in die horizontale Lage gekippt werden kann zur Aufnahme der Kontaktlinse. Anschließend kann dann der jeweilige Klappdeckel geschlossen werden. Durch Verschwenken um 180° kann dann in gleicher Weise die andere Kontaktlinse in den Kontaktlinsenträger eingelegt werden.

In den beiliegenden Figuren ist ein Ausführungsbeispiel des Reinigungsgerätes dargestellt. Anhand dieser Figuren wird die Erfindung noch näher erläutert. Es zeigt :

Fig. 1 in schematischer Darstellung im Aufriß ein Ausführungsbeispiel des Reinigungsgerätes ;

Fig. 2 eine Draufsicht in schematischer Darstellung auf die Deckeloberseite des in der Fig. 1 dargestellten Geräts und

Fig. 3 eine schematische Darstellung des Kontaktlinsenträgers.

Das dargestellte Ausführungsbeispiel des Reinigungsgerätes für Kontaktlinsen, welches als Bestandteil zu einem Kontaktlinsenpflegesatz mit einer Wasserstoffperoxidlösung zur Reinigung und Desinfizierung und einer Neutralisationslösung zur Beseitigung von Restwasserstoffperoxid an den behandelten Kontaktlinsen geeignet ist, besitzt ein Gehäuse 1, in welches nacheinander die Wasserstoffperoxidlösung und die Neutralisationslösung eingebracht werden können.

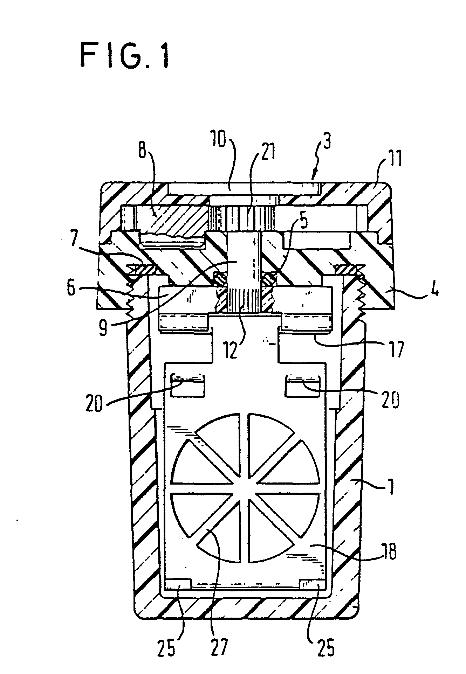

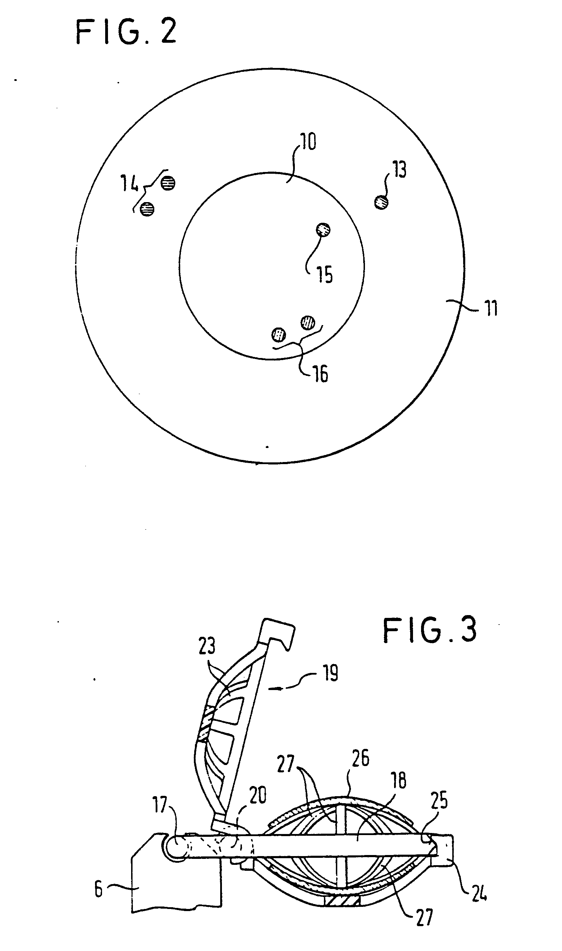

In dieses Gehäuse, welches oben offen ist, kann ein Kontaktlinsenträger 2 (Fig. 3) eingesetzt werden. In der Fig. 1 ist ein mittlerer Trägerteil 18 des Kontaktlinsenträgers 2 dargestellt. Dieser mittlere Trägerteil 18 besitzt, wie im einzelnen aus Figur 3 zu ersehen ist, nach außen gewölbte Speichen 27, zwischen die die Wasserstoffperoxidlösung bzw. Neutralisationslösung hindurchströmen kann. Auf diesen Speichen 27 kann, wie im einzelnen noch im Zusammenhang mit der Fig. 3 erläutert wird, jeweils eine Kontaktlinse 26 zu beiden Seiten des mittleren Trägerteils 18 aufgelegt werden. Am mittleren Trägerteil 18 sind zu beiden Seiten Klappdeckel 19 an Anlenkstellen 20 angelenkt. Der mittlere Trägerteil 18 ist seinerseits an einem Linsenträgerhalter 6 in einem Gelenk 17 schwenkbar gelagert.

Der Linsenträgerhalter 6 ist mit einer Welle 9 mittels einer Preßpassung 12 verbunden. Die Welle 9 ist mittels einer Dichtung 5 (O-Ring) dicht in einem Deckelunterteil 4 gelagert. Der Deckelunterteil 4 ist beispielsweise durch Verschrauben unter Zwischenlage einer Flachdichtung 7 dicht auf das Gehäuse aufgesetzt. Zwischen einem Deckeloberteil 3 und dem Deckelunterteil 4 befindet sich ein Planetengetriebe, bestehend aus einem Zahnrad 8, das mit einem drehfest auf der Welle 9 sitzenden Zahnrad 21 sowie mit einem an der Innenseite eines drehbaren Deckelteils 11 des Deckeloberteils 3 vorgesehenen Zahnkranz kämmt.

Der Deckeloberteil 3 besitzt ferner einen weiteren Deckelteil 10, der beim dargestellten Ausführungsbeispiel gegenüber dem Deckelteil 11 verdrehbar ist. Der Deckelteil 10 kann auch drehfest mit der Antriebswelle 9 verbunden sein.

Bei der Behandlung der Kontaktlinse mit einer der beiden Lösungen wird der Deckeloberteil 3 bzw. der drehbare Deckelteil 11 gegenüber dem am Gehäuse 1 festgelegten Deckelunterteil 4 gedreht. Dies kann von Hand geschehen. Bei dieser Drehung werden über den Zahnkranz an der Innenseite des Deckelteils 11 das Zahnrad 8 und das Zahnrad 21 sowie die Welle 9, der in das Gehäuse 1 ragende Kontaktlinsenträger 2 und der drehfest mit der Welle 9 verbundene Linsenträgerhalter 6 in Drehung versetzt.

Da, wie insbesondere aus der Fig. 3 zu entnehmen ist, auch die beiden am mittleren Trägerteil 18 angelenkten Klappdeckel 19 Speichen 23 aufweisen, wird einerseits eine einwandfreie Halterung der Kontaktlinsen am mittleren Trägerteil 18 und andererseits ein Hindurchströmen der jeweiligen Behandlungsflüssigkeit durch die Klappdeckel 19 hindurch gewährleistet, so daß die Behandlungsflüssigkeiten mit den Linsenoberflächen in Berührung kommen. Es wird hierbei bei der Drehung des Kontaktlinsenträgers, welche eine hin- und hergehende Drehbewegung sein kann, eine intensive Behandlung erreicht.

Wie aus der Fig. 2 zu ersehen ist, besitzt der Deckeloberteil 3 auf seinen beiden, gegeneinander verdrehbaren Deckelteilen 10 und 11 Markierungen 13, 15 und 14, 16. Die Markierungen 13 und 15 können der Wasserstoffperoxidlösung zugeordnet sein und die Markierungen 14 und 16 der Neutralisationslösung. Die Markierungen sind so vorgesehen, daß sie in zwei unterschiedlichen Relativstellungen der beiden gegeneinander verdrehbaren Deckelteile 10 und 11 einander gegenüberliegen. Diese beiden Stellungen können durch Verdrehen der Deckelteile 10 und 11 gegeneinander erreicht werden. Die beiden Deckelteile 10 und 11 sind so ausgestaltet, daß sie in den beiden angegebenen Stellungen verbleiben und nur durch Anwendung einer bestimmten Krafteinwirkung aus diesen Stellungen gebracht werden. Wenn beispielsweise die Behandlung mit der Wasserstoffperoxidlösung stattgefunden hat, können die beiden Deckelteile 10 und 11 so gegeneinander verdreht werden, daß die beiden Markierungen 13 und 15 einander gegenüberliegen, wie das in Fig. 2 dargestellt ist. Wenn die Behandlung mit der Neutralisationslösung beendet ist, können die beiden Deckelteile 10 und 11 so gegeneinander verdreht werden, daß die Markierungen 14 und 16 einander gegenüberliegen.

Beim Einlegen der Kontaktlinsen in den Kontaktlinsenhalter 2 wird der aus dem Deckelunterteil 4 und dem Deckeloberteil 3 bestehende Deckel mit seiner Oberseite auf eine Unterlage aufgesetzt und der Kontaktlinsenhalter 2 um das Gelenk 17 in eine horizontale Lage verschwenkt (Fig. 3). Der mittlere Trägerteil 18 liegt dabei waagrecht, und zum Einsetzen der Kontaktlinse 26 wird der eine Klappdeckel 19 hochgeklappt, wie das aus der Fig. 3 zu ersehen ist und die Kontaktlinse auf die speichenartigen Rippen 27 des mittleren Trägerteils 18 aufgelegt. Der Klappdeckel 19 wird heruntergeklappt, und der Kontaktlinsenträger wird um 180° verschwenkt, so daß die andere Seite des mittleren Trägerteils 18 nach oben zeigt und auf dessen speichenartige Rippen dann die andere Kontaktlinse aufgelegt wird. Auch hier wird der Klappdeckel heruntergeklappt, so daß dann beide Kontaktlinsen im Kontaktlinsenträger 2 sicher untergebracht sind. Beide Klappdeckel besitzen einen Schnappverschluß 24, der mit entsprechenden Raststellen 25 am mittleren Trägerteil in Eingriff kommen kann. Ein Öffnen beim Drehen wird verhindert. Ferner wird beim Verschließen der Klappdeckel ein Herausrutschen der Kontaktlinsen aus den dabei gebildeten Trägerkörbchen verhindert.

Im folgenden werden noch Beispiele für Bereiche der Bestandteile der Wasserstoffperoxidlösung und die Neutralisationslösung angegeben.

### Wasserstoffperoxidlösung

Wasserstoffperoxid 0,5- < 3 %
Komplexbildner 0,01-2 % Edetinsäure (Dinatriumsalz), Nitriloessigsäure
prim. Natriumphosphat 0,1-1 %
Na-diphosphat und andere Polyphosphate 0,1-5 %
Neutralelektrolyte, z. B. NaCl, KCl, LiCl, Sulfate, Phosphate 0,2 bis 2 %
eingestellt durch Phosphorsäure auf pH-Bereich 2-4

### Neutralisationslösung

Enzym (Katalasen bzw. Peroxidasen) 10-500 E/ml
Neutralelektrolyte wie Chloride, Sulfate, Phosphate, Borate, Citrate 0,2-1,5 %
Puffer wie Phosphate, Borate, Citrate 0,2-1 %
Lösliche Cellulosederivate aus der Reihe
Hydroxyethylcellulosen,
Methylcellulosen,
Methylhydroxypropyl-,
Methylhydroxyethyl-,
Hydroxypropylcellulosen
als Enzymstabilisierer 0,02-0,5 %
Bei Bedarf
geeignete, mit den Enzymen kompatible Konservierungsstoffe wie z. B.
Chlorhexidinsalze 0,5-5 mg/100 ml
Thiomersal 1-5 mg/100 ml
Sorbinsäure 0,05-0,2 %

## Patentansprüche

1. Kontaktlinsenpflegesatz, enthaltend zwei Lösungen, von denen die eine Lösung eine der Reinigung und Desinfektion der Kontaktlinse dienende Wasserstoffperoxidlösung und die andere Lösung eine enzymhaltige, der Beseitigung von Restwasserstoffperoxid von der gereinigten und desinfizierten Linse dienende Neutralisationslösung sind, dadurch gekennzeichnet, daß die Wasserstoffperoxidlösung eine 0,5 bis 3,0 %ige Lösung mit protein- oder lipoidentfernenden Zusätzen in Form von Neutralelektrolyten (NaCl, KCl, LiCl, Sulfate, Phosphate) ist und die Neutralisationslösung oberflächenaktive benetzungsfördernde Substanzen in Form von löslichen Cellulosederivaten aufweist.

2. Kontaktlinsenpflegesatz nach Anspruch 1, dadurch gekennzeichnet, daß die Wasserstoffperoxidlösung eine 0,5 bis 2 %ige Lösung ist.

3. Kontaktlinsenpflegesatz nach Anspruch 1 oder 2, dadurch gekennzeichnet, daß die Wasserstoffperoxidlösung auf einen pH-Bereich von 2-4 eingestellt ist.

4. Kontaktlinsenpflegesatz nach einem der Ansprüche 1 bis 3, dadurch gekennzeichnet, daß die Wasserstoffperoxidlösung ferner 0,01-2 % Komplexbildner, 0,1-1 % prim. Natriumphosphat und 0,1-5 % Polyphosphate enthält.

5. Kontaktlinsenpflegesatz nach Anspruch 1, dadurch gekennzeicnnet, daß die Neutralelektrolyte in einem Anteil von 0,2-2 % in der Wasserstoffperoxidlösung enthalten sind.

6. Kontaktlinsenpflegesatz nach Anspruch 1, dadurch gekennzeichnet, daß die oberflächenaktiven benetzungsfördernden Substanzen Hydroxyethyl-, Methyl-, Methylhydroxypropyl-, Methylhydroxyethyl-, Hydroxypropylcellulosen sind.

7. Kontaktlinsenpflegesatz nach Anspruch 1 oder 6, dadurch gekennzeichnet, daß die löslichen Cellulosederivate in einem Anteil von 0,02-0,5 % in der Neutralisationslösung enthalten sind.

8. Kontaktlinsenpflegesatz nach einem der Ansprüche 1 bis 7, dadurch gekennzeichnet, daß die Neutralisationslösung als Enzym Katalasen in einem Anteil von 10-500 E/ml und ferner 0,2-1 % eines Puffers und 0,2-1,5 % Neutralelektrolyte enthält.

9. Verfahren zur Pflege von Kontaktlinsen, bei dem die Kontaktlinsen zunächst zur Beseitigung von bakteriellen Verunreinigungen mit einer Wasserstoffperoxidlösung und dann zur Beseitigung von an der Kontaktlinse haftenden Restmengen an Peroxid in einer enzymhaltigen Neutralisationslösung behandelt werden, dadurch gekennzeichnet, daß eine 0,5- < 3,0 %ige Wasserstoffperoxidlösung, die protein- oder lipoidentfernende Zusätze aufweist und eine oberflächenaktive benetzungsfördernde Substanzen enthaltende Neutralisationslösung jeweils gemäß Anspruch 1 verwendet werden.

10. Verfahren nach Anspruch 9, dadurch gekennzeichnet, daß eine Wasserstoffperoxidlösung nach einem der Ansprüche 2 bis 4 und eine Neutralisationslösung nach den Ansprüchen 5 bis 7 verwendet werden.

11. Reinigungsgerät für Kontaktlinsen mit einem oben offenen Behälter zur Aufnahme einer Pflegemittellösung, einem auf die Öffnung des Behälters aufsetzbaren Deckel, welcher ein Planetengetriebe aufweist, über welches durch Drehung eines Deckelteils gegenüber dem Gehäuse ein in das Gehäuse einsetzbarer an einer Welle befestigter Kontaktlinsenträger drehbar ist, dadurch gekennzeichnet, daß für einen Kontaktlinsenpflegesatz nach einem der Ansprüche 1 bis 8 und/oder für ein Verfahren nach Anspruch 9 oder 10 der Deckeloberteil (3) in zwei gegeneinander verdrehbare Deckelteile (10 und 11) unterteilt ist, daß jeder Deckelteil (10 und 11) wenigstens eine Markierung (13, 14 und 15, 16) aufweist und daß zumindest nach der ersten Behandlung der Kontaktlinse in der Wasserstoffperoxidlösung die beiden Deckelteile (10, 11) in eine solche Stellung gegeneinander verdrehbar sind, daß wenigstens die eine Markierung (13) auf dem einen Deckelteil (11) der zugeordneten Markierung (15) auf dem anderen Deckelteil (10) gegenüber liegt.

12. Reinigungsgerät nach Anspruch 11, dadurch gekennzeichnet, daß die beiden gegeneinander verdrehbaren Deckelteile (10, 11) jeweils zwei Markierungen (13, 14 und 15, 16) aufweisen.

13. Reinigungsgerät nach Anspruch 11 oder 12, dadurch gekennzeichnet, daß der Kontaktlinsenträger (2) über einen Linsenträgerhalter (6) an der Welle (9) befestigt ist und der Linsenträgerhalter (6) ein Gelenk (17) zum Verschwenken des Kontaktlinsenträgers (2) um 180° aufweist.

14. Reinigungsgerät nach einem der Ansprüche 11 bis 13, dadurch gekennzeichnet, daß der Kontaktlinsenträger (2) zwei zur Aufnahme jeweils einer Linse geeignete Klappdeckel (22), die für das im Gehäuse (1) befindliche Lösungsmittel durchlässig sind, aufweist.

15. Reinigungsgerät nach einem der Ansprüche 11 bis 14, dadurch gekennzeichnet, daß die beiden Klappdeckel (22) an einem mittleren Trägerteil (18) angelenkt sind.

## Claims

1. Contact lens maintenance kit, containing two solutions, of which one solution is a solution of hydrogen peroxide for cleansing and disinfecting the contact lens, and the other solution is an enzyme-containing neutralisation solution for the removal of residual hydrogen peroxide from the cleansed and disinfected lens, wherein the hydrogen peroxide solution is a from 0.5 to 3.0 % solution with protein- or lipoid-removing additives in the form of neutral electrolytes (NaCl, KCl, LiCl, sulfates, phosphates) and the neutralisation solution contains surface-active wetting agents in the form of soluble cellulose derivatives.

2. The contact lens maintenance kit according to claim 1, wherein the hydrogen peroxide solution is a from 0.5 to 2 % solution.

3. The contact lens maintenance kit according to claim 1 or 2, wherein the hydrogen peroxide solution is adjusted to a pH range of from 2-4.

4. The contact lens maintenance kit according to any one of claims 1 to 3, wherein the hydrogen peroxide solution also contains 0.01-2 % complexing agents, 0.1-1 % primary sodium phosphate and 0.1-5 % polyphosphates.

5. The contact lens maintenance kit according to claim 1, wherein the neutral electrolytes are present in the hydrogen peroxide solution in a proportion of 0.2-2.0 %.

6. The contact lens maintenance kit according to claim 1, wherein the surface-active wetting agents are hydroxyethyl-, methyl-, methylhydroxypropyl-, methylhydroxyethyl-, hydroxypropyl-celluloses.

7. The contact lens maintenance kit according to claim 1 or 6, wherein the soluble cellulose derivatives are present in the neutralisation solution in a proportion of 0.02-0.5 %.

8. The contact lens maintenance kit according to any one of claims 1 to 7, wherein the neutralisation solution contains catalases as the enzyme in a proportion of 10-500 E/ml and in addition contains 0.2-1 % of a buffer and 0.2-1.5 % neutral electrolytes.

9. Method for taking care of contact lenses, in which the contact lenses are treated first with a solution of hydrogen peroxide for the removal of bacterial contamination and then in an enzyme-containing neutralisation solution for the removal of any residual amounts of peroxide adhering to the contact lens, wherein a from 0.5 to < 3.0 % solution of hydrogen peroxide containing protein- or lipoid-removing additives, and a neutralisation solution containing surface-active wetting agents, in each case according to claim 1, are employed.

10. The method according to claim 9, wherein a hydrogen peroxide solution according to any one of claims 2 to 4 and a neutralisation solution according to claims 5 to 7 are employed.

11. Cleansing equipment for contact lenses, comprising a container which is open at the top for receiving a treatment solution, a lid which may be placed over the opening of the container, which lid has planetary gears by means of which a contact lens carrier which is fastened to a shaft and can be inserted into the housing can be rotated by rotating a portion of the lid in relation to the housing, wherein for a contact lens maintenance kit according to any one of claims 1 to 8 and/or for a method according to claim 9 or 10, the upper portion (3) of the lid is divided into two opposingly rotatable lid portions (10 and 11), and each lid portion (10 and 11) carries at least one reference marking (13, 14 and 15, 16), and, at least after the first treatment of the contact lens in the hydrogen peroxide solution, the two lid portions (10, 11) may be rotated in relation to each other into such a position that at least the one marking (13) on the one lid portion (11) is opposite the marking (15) allocated to it on the other lid portion (10).

12. The cleansing equipment according to claim 11, wherein the two opposingly rotatable lid portions (10, 11) each carry two reference markings (13, 14 and 15, 16).

13. The cleansing equipment according to claim 11 or 12, wherein the contact lens carrier (2) is fastened to the shaft (9) by way of a lens-carrier support (6) and the lens-carrier support (6) has a joint (17) to allow swinging of the contact lens carrier (2) through an angle of 180°.

14. The cleansing equipment according to one of claims 11 to 13, wherein the contact lens carrier (2) has two hinged covers (22) each suitable for holding one contact lens, which are permeable to the solvent contained in the housing (1).

15. The cleansing equipment according to any one of claims 11 to 14, wherein the two hinged covers (22) are articulated to a central carrier portion (18).

## Revendications

1. Nécessaire pour l'entretien des lentilles de contact, contenant deux solutions dont l'une est une solution de peroxyde d'hydrogène servant au nettoyage et à la désinfection des lentilles de contact et l'autre est une solution neutralisante, contenant des enzymes et servant à éliminer les résidus de peroxyde d'hydrogène des lentilles nettoyées et désinfectées, caractérisé en ce que la solution de peroxyde d'hydrogène est une solution à une concentration de 0,5 à 3,0 % contenant des additifs éliminant les protéines ou les lipoïdes, à savoir des électrolytes neutres (NaCl, KCl, LiCl, sulfates, phosphates) et la solution neutralisante contient des substances tensioactives favorisant le mouillage, à savoir des dérivés solubles de la cellulose.

2. Nécessaire pour l'entretien des lentilles de contact selon la revendication 1, caractérisé en ce que la solution de peroxyde d'hydrogène est à une concentration de 0,5 à 2 %.

3. Nécessaire pour l'entretien des lentilles de contact selon la revendication 1 ou 2, caractérisé en ce que la solution de peroxyde d'hydrogène est réglée à un pH dans l'intervalle de 2 à 4.

4. Nécessaire pour l'entretien des lentilles de contact selon l'une des revendications 1 à 3, caractérisé en ce que la solution de peroxyde d'hydrogène contient en outre de 0,01 à 2 % d'agents complexants, de 0,1 à 1 % de phosphate monosodique et de 0,1 à 5 % de polyphosphates.

5. Nécessaire pour l'entretien des lentilles de contact selon la revendication 1, caractérisé en ce que les électrolytes neutres sont contenus en proportions de 0,2 à 2 % dans la solution de peroxyde d'hydrogène.

6. Nécessaire pour l'entretien des lentilles de contact selon la revendication 1, caractérisé en ce que les substances tensio-actives favorisant le mouillage consistent en hydroxyéthylcellulose, méthylcellulose, méthylhydroxypropylcellulose, méthylhydroxyéthylcellulose, hydroxypropylcellulose.

7. Nécessaire pour l'entretien des lentilles de contact selon la revendication 1 ou 6, caractérisé en ce que les dérivés solubles de la cellulose sont contenus en proportions de 0,02 à 0,5 % dans la solution neutralisante.

8. Nécessaire pour l'entretien des lentilles de contact selon l'une des revendications 1 à 7, caractérisé en ce que la solution neutralisante contient en tant qu'enzyme une catalase en proportions de 10 à 500 U/ml et en outre 0,2 à 1 % d'un tampon et 0,2 à 1,5 % d'électrolytes neutres.

9. Procédé pour l'entretien des lentilles de contact dans lequel on traite les lentilles de contact d'abord par une solution de peroxyde d'hydrogène pour élimination des impuretés bactériennes puis par une solution neutralisante contenant des enzymes pour l'élimination des résidus de peroxyde adhérant aux lentilles de contact, caractérisé en ce que l'on utilise une solution de peroxyde d'hydrogène à une concentration de 0,5 à moins de 3,0 % contenant des additifs éliminant les protéines et les lipoïdes selon la revendication 1 et une solution neutralisante contenant des additifs tensio-actifs favorisant le mouillage selon la revendication 1.

10. Procédé selon la revendication 9, caractérisé en ce que l'on utilise une solution de peroxyde d'hydrogène selon l'une des revendications 2 à 4 et une solution neutralisante selon les revendications 5 à 7.

11. Appareil de nettoyage pour lentilles de contact comprenant un récipient ouvert à sa partie supérieure pour le logement d'une solution de produit de nettoyage, un couvercle pouvant être posé sur l'ouverture du récipient, cet appareil comprenant un mécanisme planétaire par l'intermédiaire duquel, par rotation d'une partie de couvercle par rapport au logement, on peut faire tourner un support de lentilles de contact solidaire d'un axe et pouvant être placé dans le logement, caractérisé en ce que, pour un nécessaire pour l'entretien des lentilles de contact selon l'une des revendications 1 à 8 et/ou pour un procédé selon la revendication 9 ou 10, la partie supérieure de couvercle 3 est divisée en deux parties de couvercle 10 et 11 pouvant pivoter l'une par rapport à l'autre, chacune des parties de couvercle 10 et 11 porte au moins une marque 15, 14 et 15, 16, et, au moins après le premier traitement de la lentille de contact dans la solution de peroxyde d'hydrogène, les deux parties de couvercle 10, 11 peuvent être déplacées par rotation l'une par rapport à l'autre et amenées dans une position telle qu'au moins une marque 13 d'une partie de couvercle 11 fait face à la marque correspondante 15 de l'autre partie de couvercle 10.

12. Appareil de nettoyage selon la revendication 11, caractérisé en ce que les deux parties de couvercle 10, 11 pouvant pivoter l'une par rapport à l'autre portent chacune deux marques 13, 14 et 15, 16.

13. Appareil de nettoyage selon la revendication 11 ou 12, caractérisé en ce que le support de lentilles de contact 2 est fixé sur un appui de support de lentilles 6 à l'axe 9 et l'appui de support de lentilles 6 porte une articulation 17 permettant un pivotement de 180° du support de lentilles de contact 2.

14. Appareil de nettoyage selon l'une des revendications 11 à 13, caractérisé en ce que le support de lentilles de contact 2 porte deux couvercles pivotants 22 pouvant chacun accepter une lentille et qui sont perméables pour le solvant qui se trouve dans le logement 1.

15. Appareil de nettoyage selon l'une des revendications 11 à 14, caractérisé en ce que les deux couvercles pivotants 22 sont articulés sur une partie médiane de support 18.

# FIG.1

FIG. 2

FIG. 3